# EUROPEAN PATENT APPLICATION

(11) **EP 4 424 248 A1**
(43) Date of publication of application: **04.09.2024**
(21) Application number: 23159745.1
(22) Date of filing: 02.03.2023
(51) Int. Cl.: A61B 17/04, A61B 17/06, A61B 17/062

(54) **SURGICAL DEVICE HAVING A FELTING NEEDLE WITH A BLUNT TIP**

(71) Applicant: ZuriMED Technologies AG, 8008 Zürich (CH)
(72) Inventor: BRUN, Sascha, 8006 Zurich (CH); BANZET, Pol, 8006 Zürich (CH); LI, Xiang, 8610 Uster (CH); BACHMANN, Elias, 8049 Zurich (CH); MÖHL, Andrea Gabriela, 5600 Lenzburg (CH); SENN, Ronja, 5436 Würenlos (CH)
(74) Representative: Vossius & Partner Patentanwälte Rechtsanwälte mbB

(57) **Abstract**

The present invention relates to a surgical device (1) for attaching a non-woven textile to human or animal soft tissue by carrying individual fibers of the non-woven textile into the human or animal soft tissue. The device comprises a holder (3, 5) and a felting needle (2, 31, 51, 61, 71, 81, 91). The holder holds the felting needle. The felting needle is movable is reciprocally between the first and the second position relatively to the holder. The felting needle has a longitudinal axis, a proximal end, and a distal end. The felting needle is configured to push individual fibers of the non-woven textile into the human or animal soft tissue. The distal end of the felting needle has a blunt tip for catching and pushing fibers of the novel textile with a distal end surface on the blunt tip.

## Description

The present application relates to a surgical device for attaching a non-woven textile to human or animal soft tissue by carrying individual fibers of the non-woven textile into the human or animal soft tissue. The invention also relates to a set including the surgical device, and a method of attaching a non-woven textile to human or animal soft tissue by carrying individual fibers of a non-woven textile into the human or animal soft tissue.

The present applicant recently disclosed the Surgical FiberLock Technology (see e.g. PCT/CH2019/000015) as a method and device for implanting a dedicated biocompatible implant made of non-woven microfibers using a dedicated device. This innovative technology allows for the distribution of microfibers throughout soft tissue to repair it and augment its repairs, while also minimizing the occurrence of suture cut through, which is a common issue resulting in revision of repairs using traditional suturing. Additionally, this technology has the potential to reinforce and repair severely damaged or weak tissue that could not be repaired with traditional suturing.

The Surgical FiberLock Technology implantation process involves the utilization of a high frequency reciprocating needle. The needle penetrates both, an implant comprising a non-woven textile and human or animal soft tissue and is designed with features that allow it to carry the implant fibers into the soft tissue. As a result, the implant becomes securely and evenly anchored in the tissue.

The previously described needles have barbs positioned on the edges of the needle body, which are intended to hook onto the microfibers of the implant. However, one limitation of these barbs is the distance to the piercing tip of the needle from the fiber carrying feature. As a result, the needle has to penetrate the tissue beyond the depth required for fiber carriage. This creates a hazard, especially in situations where the soft tissue is in proximity to sensitive areas, such as nerves or major circulatory organs, e.g., in cardiovascular tissue.

The process of felting individual fibers of a non-woven textile is known in other technical areas such as the textile industry. There, felting needles usually include barbs along the circumference of the needle are used to catch fibers and to draw them into another textile. Such felting needles allow for a consistent felting, in particular for consistently felting two textiles over large areas with an even distribution of carried fibers. While the felting needles used in the textile industry may have a similar general shape as such, they deviate in a number of aspects. When felting non-woven textiles to biomedical tissues, a different set of challenges arise. In particular, human or animal soft tissues are often close to hard tissues, only a relatively small area is felted, and in the small area more fibers need to be felted. In addition, traditional felting needles require penetration beyond the two tissues that are felted to each other in order to push the fibers sufficiently far and are complex to manufacture.

It is the objective technical problem to overcome one or more of the above disadvantages of the prior art. In particular, it is the objective technical problem to provide a surgical device for attaching a non-woven textile to human or animal soft tissue by carrying individual fibers of the non-woven textile into the human or animal soft tissue that causes a strong bond between the textile and the tissue.

The objective problem is solved by the features of the independent claims. One aspect of the present invention relates to a surgical device for attaching a non-woven textile to human or animal soft tissue by carrying individual fibers of the non-woven textile into the human or animal soft tissue. The device comprises a holder and a felting needle. The holder holds the felting needle. The felting needle is movable reciprocally between a first position and a second position relatively to the holder. The felting needle has a longitudinal axis, a proximal end, and a distal end. The felting needle is configured to push individual fibers of the non-woven textile into the human or animal soft tissue. The distal end of the felting needle has a blunt tip for catching and pushing fibers of the novel textile with a distal end surface on the blunt tip.

Contrary to traditional felting needles, the felting needle of the independent claims is configured to push the fibers with the distal end, rather than with barbs that are arranged in a proximal position. This allows to push the fibers as far as the felting needle penetrates. The applicant found that such felting needles provide for a better attachment than traditional felting needles for the same needle penetration depth when attaching non-woven textile to human or animal soft tissue. Traditional felting needles known from the textile industry have a sharp tip. The sharp tip allows the traditional felting needles known from the textile industry to penetrate a non-woven textile without carrying any fibers at the distal end portion. Also, the sharp tip may reduce the penetrating force into the fabric. In contrast, the tip of the felting needles disclosed herein are blunt and includes a distal end surface that catches at least one fiber when passing through a non-woven textile.

A further advantage may be that less tissue is damaged since the felting needle does not need to penetrate as far. Further, the distance between the first and second positions is reduced, and the amplitude of the reciprocal movement is reduced, which may allow for higher frequencies and/or lower needle speeds during the reciprocal motion.

Felting as used herein may be understood as an intertwining and/or entangling of tissue fibers and implant fibers. The intertwining and/or entangling is achieved by carrying, e.g., pushing, individual or a few fibers of the non-woven textile into the human or animal soft tissue with a felting needle.

The reciprocal motion may be understood as a back-and-forth motion. For example, the needle is moved along a line or rotated around an axis about an angle. The non-woven material may be or form part of an implant. The implant may be provided with the surgical device. Example materials and example implants are shown for example in PCT/CH2019/000015, PCT/EP2020/081887, PCT/EP2020/081891, EP 22177290.8, and PCT/EP2020/081881. The implants may comprise a drug as described in EP 22177290.8.

The holder may have a tubular member that guides that felting needle along the reciprocal movement. The surgical device may also include a driving member for moving the felting needle reciprocally between a first and second position. A distance between the first and second positions may be 1-30 mm, more preferably 6-15 mm. In particular examples, the distance is 6.5 mm, 8.5 mm, or 12 mm. The at least one felting needle may have a length of at least the thickness of an implant. In some embodiments, the needle may have a length of at least 4, 6, 8, or 10 mm. The motor may actuate the needle with a frequency of 1-200 Hz, preferably 10-100 Hz, most preferably 20-80 Hz and in one embodiment 40 Hz. In general, the holder may be designed as shown in any of the following applications: PCT/CH2019/000015, PCT/EP2020/081887, PCT/EP2020/081891, EP 22177290.8, and PCT/EP2020/081881

Blunt as used herein may describe a tip that has at least some area at the distal end that is suitable to push individual or several fibers from the non-woven into the soft tissue. The area may have a normal direction along the longitudinal axis or may be at an angle of 45° or less to the longitudinal axis.

In a preferred embodiment, the distal end surface is even and extends substantially perpendicular to the longitudinal axis of the felting needle. A perpendicular end surface is particularly suited for carrying fibers, since the fibers are caught particularly well by the surface without slipping off.

In a particularly preferred embodiment, the felting needle has the shape of a flat head screwdriver. This embodiment is particularly simple and easy to manufacture and allows for a strong entanglement between the fibers of the soft tissue and the non-woven textiles. Other shapes that have a blunt tip such as a blunt Phillips screwdriver (cross shape), Torx screwdriver (star shape) or a hexagon screwdriver are also suitable.

In a further embodiment, the distal end surface is even and extends at an angle of more than 45°, 60°, 70° or 80° to the longitudinal axis of the felting needle. Thus, the flat surface may also be angled. The applicant noticed that angled surfaces can still carry fibers. In particular, the distal end surface may have a roughness as described below that allows for carrying the fibers.

In a preferred embodiment, the blunt tip is circular, rectangular, cross -shaped, starshaped, 8-shaped or elliptic. These shapes refer to the shape of the distal tip when seen from the distal side or normal to the distal end surface.

In a preferred embodiment, the distal end surface comprises one, two, three or more notches. The notches may have a width of 0.005-0.1 mm. The notches help in catching and carrying individual fibers and carrying these fibers into the soft tissue. Individual fibers may mean that one or few (e.g., two or three or four) fibers are carried. The notches may be arranged in parallel or at any angle to each other. The notches may be straight, have a wave shape, may be angled, or curved. The notches may extend in the direction of a width or depth of the distal end surface or anything between. For example, the notches may extend along a direction parallel or perpendicular to the longest dimension of distal end surface. The notches may have a depth of up to of 0.005-0.1 mm. The notches may have a tapering shape, and, in particular, they may be semicircular, V-shaped, or U-shaped.

In a preferred embodiment, the blunt tip at the distal end has a first width perpendicular to the longitudinal axis of the felting needle of at most 1 mm. In a preferred embodiment, the first width is at most 0.5 mm. Particularly preferred, the first width is at most 0.4 mm. Most preferred, the first width is at most 0.3 mm. These dimensions are particularly suitable for catching fibers of the non-woven textile and carrying the fibers into soft tissue. As mentioned above, these fibers then become entangled with the soft tissue. If the distal end of the felting needle is too large, too many fibers are caught at the same time and pushed towards the soft tissue. Rather than catching few fibers or only a single fiber that can be drawn out of the felt alone, this simply results in pushing too many fibers and damages the soft tissue.

In a further embodiment, the first width is at least 0.02, 0.03, 0.04 or 0.05 mm. These minimal widths ensure that the fibers can be caught by the blunt tip. If the first width is less, then the felting needle may penetrate the felt without catching any or only rarely fibers, resulting in no or poor attachment. Further, if the first width is below these values, the felting needle may cut the fibers in some cases.

The blunt tip may also have a second width that is perpendicular to the longitudinal axis of the felting needle and perpendicular to the first width. The second with may be at least 0.01, 0.02, 0.03, 0.04, or 0.05 mm. Similar consideration as for the first width apply.

In a preferred embodiment, the felting needle comprises a distal end portion. The blunt tip may form the distal end of the distal end portion. The distal end portion includes the blunt tip. The distal end portion may taper towards the distal end. The distal end portion may be conical. In preferred examples, the distal end portion is frustoconical. The tapering and in particular the conical shape may help the distal end portion of the felting needle in moving through the textile and/or the soft tissue and catching only individual fibers.

In a preferred embodiment, the distal end portion has a mean line roughness of 3.2, 2.5, 2.0, 1.5, 1.0 µm or more. Mean line may be understood as the deviation of a surface from a mean height. The roughness may be the arithmetic average value of a roughness profile determined from deviations about the center line within the evaluation length. The roughness of the distal end portion and in particular the roughness of the distal end surface may support catching and carrying individual fibers.

In a preferred embodiment, the felting needle does not comprise barbs. In particular, there may be no barbs along the outer mantle surface of the felting needle. The felting needle claimed herein carries the individual fibers with the distal end surface of the blunt tip. Thus, the felting needle does not need to comprise barbs. This provides for a simpler, easier to manufacture felting needle.

In a preferred embodiment, the distal end surface is recessed, in particular rounded, towards the proximal direction for catching fibers. This allows for efficiently catching not just a single fiber, but rather catching multiple fibers at once.

In a further embodiment, the device additionally comprises a motor. The motor is configured to drive the felting needle back and forth between first and second positions. Thereby, the motor provides the reciprocal motion of the felting needle.

A further aspect of the present invention is directed towards a set. The set includes a surgical device as described above and an implant that comprises a non-woven textile.

A further aspect of the present invention is directed to a method of attaching an implant comprising a non-woven textile to human or animal soft tissue by carrying individual fibers of the non-woven textile into the human or animal soft tissue. The method includes the following steps:
- Providing a set according as described above;
- Positioning the non-woven textile adjacent to the soft tissue;
- Actuating the felting needle to move back and forth between the first and second positions, wherein the felting needle moves through the non-woven textile and into the soft tissue; and
- Pushing fibers of the non-woven textile, with the blunt tip, from the non-woven textile into the soft tissue.

Non-limiting embodiments of the invention are described, by way of example only, in the accompanying drawings, in which:
- Figure 1:: shows a surgical device as known in the art.
- Figure 2:: shows a felting needle as used in the surgical device of figure 1.
- Figures 3A and 3B:: show a first embodiment of a felting needle according to the invention in a top view.
- Figures 4A and 4B:: show the first embodiment of a felting needle according to the invention in a perspective view and in a side view.
- Figures 5A and 5B:: show a second embodiment of a felting needle according to the invention.
- Figures 6A and 6B:: show a third embodiment of a felting needle according to the invention.
- Figures 7A to 7C:: show further embodiments of felting needles according to the invention.

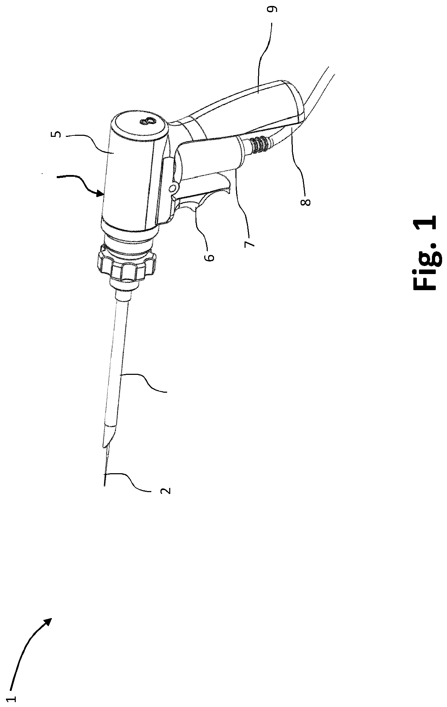

Figure 1 shows a perspective view of a surgical device 1 as disclosed in WO 2022/100833 (ZURIMED TECHNOLOGIES AG). The surgical device 1 includes a holder that comprises a housing 5 and a guide tube 3 extending from the housing 5. A felting needle 2 is arranged within the guide tube 3 and extends from the end of the guide tube 3. The felting needle 2 can be moved reciprocally forwards and backwards along its axis and the axis of the guide tube 3. The surgical device 1 includes a handle 9 with a trigger lever 6. Further, the surgical device 1 is connected to an energy source with a power cord 8 that connects to the surgical device 1. An actuator -here electrical motor 7 - is arranged within the housing 5 of the surgical device 1. The motor 7 drives the reciprocal motion of the needle 2. When a user pulls the trigger lever 6, the electrical motor 7 moves the felting needle 2. The felting needle moves back and forth along its longitudinal axis with a frequency of about 40 Hz with an amplitude of 12 mm in this particular embodiment (other frequencies and amplitudes are possible). Such a surgical device is disclosed in detail in WO 2022/100833.

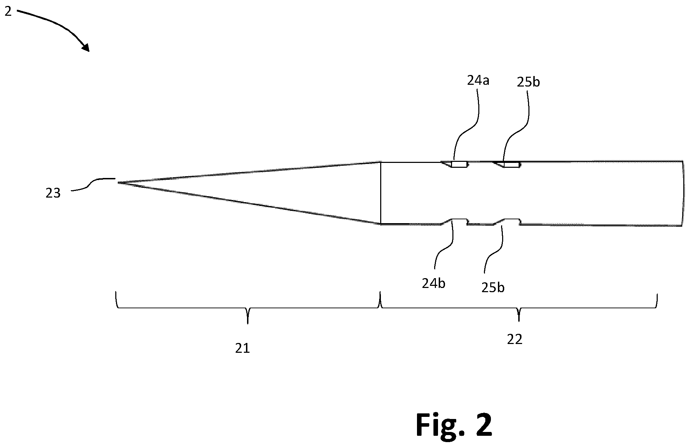

The felting needle 2 of the surgical device 1 is shown in figure 2. The felting needle 2 has a cylindrical portion 21 and a tip portion 22. The tip portion 22 tapers conically towards a sharp tip 23. The cylindrical portion 21 includes barbs 24a-b and 25 a-b. The barbs 24a-b and 25 a-b catch fibers in non-woven textiles when the felting needle moves through the textile.

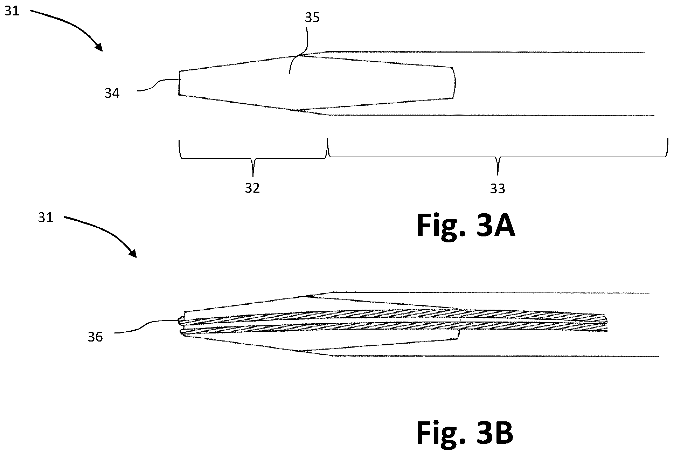

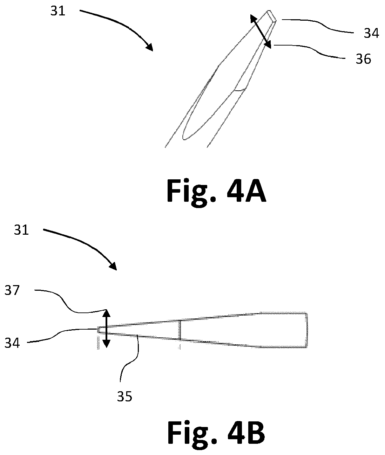

Instead of using a felting needle with a sharp tip as shown in figure 2, the present invention is directed at providing a felting needle with a blunt tip. One example of such a felting needle is shown in figures 3A to 4B. Figures 3A to 4B all show a felting needle 31 that can replace the felting needle 2 in the surgical device 1. The felting needle 31 includes a cylindrical portion 33 and a tip portion 32. The tip portion 32 tapers conically. In the shown example, the cylindrical portion 33 is circular cylindrical but may also be rectangular, quadratic, hexagonal, elliptic, trapezoidal or any other cylindrical shape. Contrary to the felting needle 2, the cylindrical portion 33 does not include barbs. Rather, the tip portion 32 is blunt and includes a distal end surface 34 that is flat. The felting needle also includes a flat angled surface 35 that extends from the cylindrical portion to the distal end surface 34. The flat angled surface 35 may have an angle of about 5° with respect to the longitudinal axis of the felting needle 31. The angled surface 35 extends in the tip portion as well as in the cylindrical portion 33. Due to the flat angled surface 35 and the conical tapering in the tip portion 32, a rectangular distal end surface 34 results (see perspective view of figure 4A). As a result, the felting needle 31 as shown in figures 3A to 4B has generally the shape of a flat head screwdriver. The rectangular distal end surface catches individual fibers 36 as shown in figure 3B and pushes them into soft tissue.

In the shown embodiment, the end surface 34 is rectangular and may have a first width of 0.13 mm along a first width direction 36 (see figure 4A). The rectangular end surface may have a second width 0.05 mm along a second width along a second width direction 37 (see figure 4B). The second width direction 37 is perpendicular to the first width direction 36. As described above, the width measurements may vary.

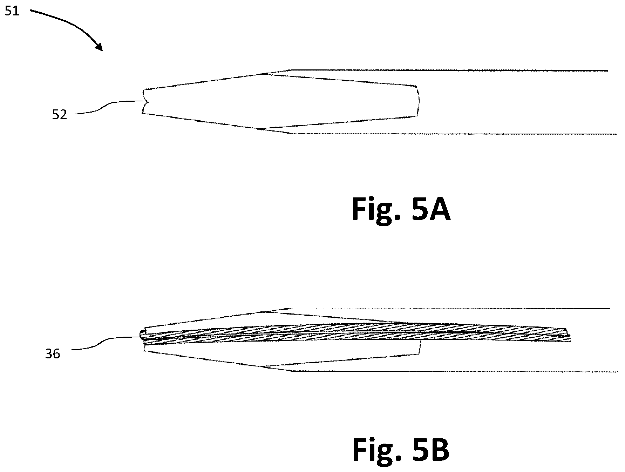

Figures 5A and 5B show a second embodiment of a felting needle 51 according to the invention. The felting needle 51 is largely similar to the felting needle 31 shown in figures 3A to 4B. In addition, the felting needle 51 includes a notch 52 at its distal end surface. The notch extends along the direction of the second width and is substantially V-shaped. The V-shaped notch 52 is typically adapted to the thickness of the fibers of a textile to be felted. Such fibers may have a thickness between 5-200 µm. The notch has similar or slightly larger dimensions. In particular, the notch has a width of 5 to 100 µm.

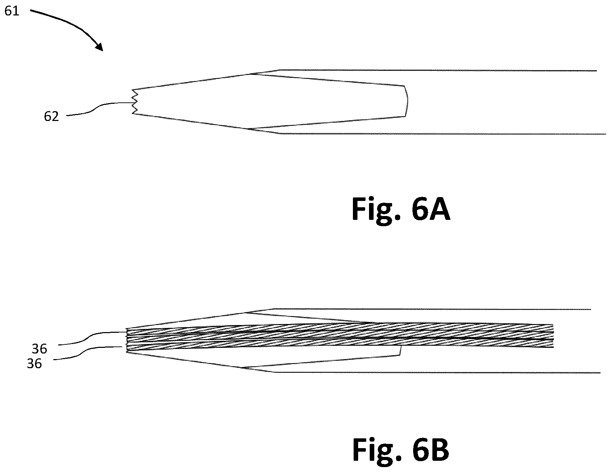

Figures 6A and 6B show a third embodiment of a felting needle 61 according to the invention. The felting needle 61 is largely similar to the felting needle 51 shown in figures 5A and 5B. Instead of only one notch, the felting needle 51 includes multiple notches 62 at its distal end surface. This allows the felting needle to catch multiple fibers 36 more securely, as shown in figure 6B. Similarly to the notch 52, the notches 62 extend generally in the direction of the second width. Any of the shown other shown embodiments may additionally comprise one or more notches. In other embodiments the notches could extend along the first width. The first width may be larger than the second width.

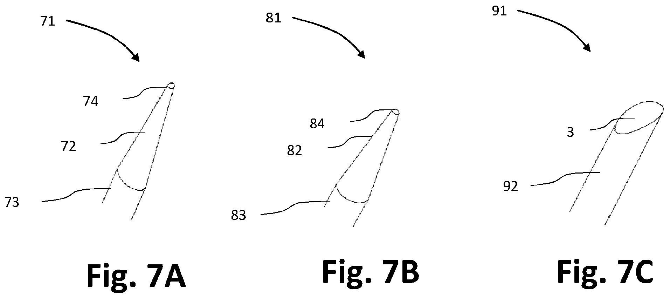

Figure 7A shows a fourth embodiment of a felting needle 71 with a blunt tip. Similarly to the felting needles described above, the felting needle 71 includes a cylindrical portion 73 and a tapering portion 72. The tapering portion 72 has a frustoconical shape such that an even distal end surface 74 is formed at the tip. The distal end surface is substantially perpendicular to the longitudinal axis of the felting needle.

Figure 7B shows a fifth embodiment of a felting needle 81 with a blunt tip. The felting needle 81 is similar to the felting needle 71 shown in figure 7A and includes a cylindrical portion 83 and a tapering portion 82 with a frustoconical shape and an even distal end surface 84. Contrary to the fourth embodiment, the even distal end surface 84 extends at an angle of 80° to the longitudinal axis of the felting needle.

Figure 7C shows a sixth embodiment of a felting needle 91. Contrary to the previously shown felting needles, the felting needle 91 only includes a cylindrical portion 92 and no tapering portion. The cylindrical portion 92 includes a distal end surface 93. The distal end surface may extend at an angle of 90° to the longitudinal axis of the felting needle 91. Then, the felting needle 91 forms a stamp that drives individual fibers into the soft tissue. In the shown embodiment, the distal end surface 93 extends at an angle of 70° to the longitudinal axis of the felting needle 91 and has an average roughness of 1.5.

The felting needles as shown herein are manufactured similarly to the known needles as shown e.g., in WO 2022/100833. However, instead of forming barbs, a sharp tip may be removed using e.g., EDM (electrical discharge machining), micro machining techniques, grinding, electro-grinding or laser grinding.

## Claims

1. A surgical device (1) for attaching a non-woven textile to human or animal soft tissue by carrying individual fibers of the non-woven textile into the human or animal soft tissue, the device comprising:
a holder (3, 5) and a felting needle (2, 31, 51, 61, 71, 81, 91); the felting needle (2, 31, 51, 61, 71, 81, 91) being held by the holder (3, 5),
wherein the felting needle (2, 31, 51, 61, 71, 81, 91) is movable reciprocally between a first and a second position relatively to the holder,
wherein the felting needle (2, 31, 51, 61, 71, 81, 91) has a longitudinal axis, a proximal end, and a distal end, the felting needle (2, 31, 51, 61, 71, 81, 91) being configured for pushing individual fibers of the non-woven textile into the human or animal soft tissue,
**characterized in that** the distal end of the felting needle (2, 31, 51, 61, 71, 81, 91) has a blunt tip for catching and pushing the fibers of the non-woven textile with a distal end surface (34, 74, 84, 93) on the blunt tip.

2. Surgical device according to claim 1, wherein the distal end surface (34, 74, 84, 93) is even and extends substantially perpendicularly to the longitudinal axis of the felting needle (2, 31, 51, 61, 71, 81, 91).

3. Surgical device according to claim 2, wherein the felting needle (2, 31, 51, 61, 71, 81, 91) has a shape of a flat head screwdriver.

4. Surgical device according to claim 1, wherein the distal end surface (34, 74, 84, 93) is even and extends at an angle of more than 45°, 60°, 70°, or 80° to the longitudinal axis of the felting needle (2, 31, 51, 61, 71, 81, 91).

5. Surgical device according to one of the preceding claims, wherein blunt tip is circular, rectangular, cross-shaped, 8-shaped or elliptic.

6. Surgical device according to one of the preceding claims, wherein the distal end surface (34, 74, 84, 93) comprises one, two, three or more notches.

7. Surgical device according to one of the preceding claims, wherein the blunt tip at the distal end has a first width perpendicular to the longitudinal axis of the felting needle (2, 31, 51, 61, 71, 81, 91) of at most 3 mm, 1 mm, preferably at most 0.5 mm, more preferred at most 0.4 mm, most preferred at most 0.3 mm, or 0.2 mm.

8. Surgical device according to one of the preceding claims, wherein the blunt tip has a first width perpendicular to the longitudinal axis of the felting needle (2, 31,51, 61, 71, 81, 91) of at least 0.02, or 0.03 mm, preferably at least 0.04 mm, more preferred at least 0.05 mm.

9. Surgical device according to one of the preceding claims, wherein the distal end has a second width perpendicular to the longitudinal axis of the felting needle (2, 31, 51, 61, 71, 81, 91) and perpendicular to the first width of at least 0.01 mm, preferably at least 0.02 mm, more preferred at least 0.03 mm, most preferred at least 0.04 mm or 0.05 mm.

10. Surgical device according to one of the preceding claims, wherein the felting needle (2, 31, 51, 61, 71, 81, 91) comprises a distal end portion that includes the blunt tip, wherein the distal end portion tapers towards the blunt tip, and wherein the distal end portion is preferably conical.

11. Surgical device according to one of the preceding claims, wherein a distal end portion has a mean line roughness of 3.2, 2.5, 2.0, 1.5, 1.0 µm or more.

12. Surgical device according to one of the preceding claims, wherein the felting needle (2, 31, 51, 61, 71, 81, 91) does not comprise barbs.

13. Surgical device according to one of the preceding claims, the device additionally comprising a motor (7), the motor (7) being configured to drive the felting needle (2, 31, 51, 61, 71, 81, 91) back and forth between the first and second positions.

14. Set including the surgical device (1) according to one of the preceding claims and an implant comprising a non-woven textile .

15. Method of attaching an implant comprising a non-woven textile to human or animal soft tissue by carrying individual fibers of the non-woven textile into the human or animal soft tissue, comprising the following steps:
- Providing a set according to claim 14;
- Positioning the non-woven textile adjacent to the soft tissue;
- Actuating the felting needle (2, 31, 51, 61, 71, 81, 91) to move back and forth between the first and second positions, wherein the felting needle (2, 31, 51, 61, 71, 81, 91) moves through the non-woven textile and into the soft tissue; and
- Pushing fibers of the non-woven textile, with the blunt tip, from the non-woven textile into the soft tissue.
